# FASCICULE DE BREVET EUROPEEN

(11) **EP 0 938 318 B1**
(45) Date de publication et mention de la délivrance du brevet: **02.05.2001**
(21) Numéro de dépôt: 97937651.4
(22) Date de dépôt: 22.08.1997
(51) Int. Cl.: A61K 31/505, A61K 9/20

(54) **COMPRIME A LIBERATION CONTROLEE DE CHLORHYDRATE D'ALFUZOSINE**
TABLETTE MIT GESTEUERTER FREISETZUNG VON ALFUZOSINHYDROCHLORID
TABLET WITH CONTROLLED RELEASE OF ALFUZOSINE CHLORYDRATE

(30) Priorité: 29.08.1996 FR 9610551; 10.04.1997 FR 9704386
(43) Date de publication de la demande: 01.09.1999
(73) Titulaire: SANOFI-SYNTHELABO, 75013 Paris (FR); JAGOTEC AG, 6502 Hergiswil (CH)
(72) Inventeur: MAGGI, Lauretta, I-27100 Pavia (IT); CONTE, Ubaldo, I-20052 Busto Arisizio (IT); GRENIER, Pascal, F-68300 Saint Louis (FR); VERGNAULT, Guy, F-68300 Saint Louis (FR); DUFOUR, Alain, F-75007 Paris (FR); JARREAU, François, Xavier, F-78000 Versailles (FR); RAUCH-DESANTI, Clémence, F-77330 Ozoire la Ferrière (FR)
(74) Mandataire: Thouret-Lemaitre, Elisabeth
(86) Numéro de dépôt international: FR9701515
(87) Numéro de publication internationale: WO9808515

(56) Documents cités:
- EP-A- 0 204 597
- EP-A- 0 673 650
- WO-A-94/27582

## Description

La présente invention a pour objet un comprimé à libération contrôlée de chlorhydrate d'alfuzosine et une composition pharmaceutique contenant un ou plusieurs comprimés.

Le chlorhydrate d'alfuzosine est une substance active connue dans le traitement de l'hypertrophie bénigne de la prostate. Les données et les expérimentations concernant l'activité du produit sont nombreuses. En particulier, il y a de nombreuses données concernant la disponibilité biologique du produit et la pharmacocinétique de la substance active. Il s'agit en effet d'une substance active qui présente un temps de demi-vie relativement court et une absorption plus intense au niveau du duodénum-jéjunum mais dont l'importance diminue le long du tractus intestinal. De ce fait, pour un effet optimum, l'administration de chlorhydrate d'alfuzosine par comprimés traditionnels (à désagrégation rapide et dissolution) doit se faire plusieurs fois par jour. Pour ces raisons, le chlorhydrate d'alfuzosine est un candidat à la réalisation d'une préparation pharmaceutique à libération contrôlée dans les parties hautes proximales du tractus (duodénum et jéjunum).

Dans le secteur pharmaceutique des progrès remarquables ont été obtenus, ces dernières années, dans la réalisation de systèmes de libération de substances actives de plus en plus perfectionnés et capables de libérer les substances actives véhiculées en eux selon des cinétiques et des modalités de libération conçues pour permettre des effets thérapeutiques optimaux.

Les formes à libération prolongée (ou préparations retard) sont caractérisées en ce qu'elles véhiculent une quantité de médicament nettement supérieure aux préparations pharmaceutiques traditionnelles de manière à permettre une simplification au plan posologique. C'est-à-dire que l'on passe de deux, trois ou plusieurs administrations par jour à une seule administration d'une préparation pharmaceutique (ou système thérapeutique) capable de satisfaire la couverture thérapeutique toute la journée.

Des réalisations de ce genre sont utilisées et commercialisées depuis longtemps, parmi celles-ci il faut mentionner : les chronoïdes, les microcapsules et micro-matrices, les comprimés génériquement définis "retard", les comprimés gastro-résistants et des réalisations plus complexes telles que les matrices hydrophiles qui s'érodent et/ou gonflent. Récemment des systèmes thérapeutiques plus raffinés ont été réalisés, par exemple les systèmes dits à "réservoir", les systèmes Geomatrix® tels que décrits dans les brevets US 4.839.177 et 5.422.123.

La plupart de ces nouveaux systèmes thérapeutiques sont capables de libérer la substance active véhiculée en eux, à une vitesse constante (c'est-à-dire selon une cinétique d'ordre zéro) jusqu'à la libération complète de la substance active, indépendamment des conditions de pH du tractus gastro-intestinal, et ainsi de façon uniforme le long du tractus gastro-intestinal. Il en résulte que ces systèmes peuvent être largement appliqués en cas d'administration de médicaments qui sont absorbés d'une façon uniforme dans le tractus gastro-intestinal. Cependant ces systèmes pharmaceutiques peuvent présenter des inconvénients majeurs au cas où on véhiculerait en eux des substances actives, telles que l'alfuzosine, ayant une absorption plus intense au niveau du duodénum-jéjunum diminuant ensuite dans le tractus. Dans ce cas, en effet, seulement une quantité très limitée de la substance active véhiculée peut être absorbée et exercer donc l'activité thérapeutique souhaitée, tandis que la plus grande partie du médicament libérée par la préparation pharmaceutique ne peut pas être absorbée, car dans des portions plus terminales du tractus gastro-intestinal, les barrières biologiques sont peu capables de permettre le passage du médicament.

La présente demande de brevet a pour objet un comprimé à libération contrôlée de chlorhydrate d'alfuzosine remédiant aux inconvénients mentionnés plus haut.

L'invention consiste en un comprimé pharmaceutique à deux ou trois couches, caractérisé en ce qu'il a la structure suivante :
a) une première couche 1 ayant la propriété de gonfler remarquablement et rapidement au contact avec les liquides biologiques aqueux, ladite couche étant produite par compression d'un mélange ou d'un granulé comprenant des polymères hydrophiles constituant de 5,0 à 90 % et de préférence de 10 à 85 % du poids de la couche,
b) une deuxième couche 2 adjacente ou superposée à la première couche, dans laquelle le chlorhydrate d'alfuzosine est véhiculé, cette couche étant formulée avec des polymères hydrophiles et avec d'autres substances auxiliaires, afin de donner à la préparation des propriétés de compressibilité convenables et pour permettre la libération de chlorhydrate d'alfuzosine dans un intervalle de temps prédéterminé,
c) et éventuellement une troisième couche 3 obtenue par compression et appliquée sur la couche 2, généralement constituée en particulier par des polymères hydrophiles qui gélifient et/ou gonflent puis peuvent éventuellement s'éroder et ayant fonction de barrière modulant la libération du chlorhydrate d'alfuzosine de la couche 2, la couche 3 étant d'abord très peu perméable au passage de la substance active.

L'invention est caractérisée par le fait qu'au contact avec le suc gastrique, à la suite d'un gonflement rapide et remarquable d'au moins une des couches 1 ou 3, ainsi que par l'éventuel gonflement de la couche 2, la préparation pharmaceutique augmente de volume de façon remarquable ; ainsi la préparation pharmaceutique reste plus longtemps au niveau de l'estomac. De cette façon, la plus grande partie du chlorhydrate d'alfuzosine contenue, pourra être absorbée de façon contrôlée dans la portion du tractus gastro-intestinal où la capacité d'absorption est la plus élevée.

Les couches 1 et 3 peuvent avoir une composition identique et des propriétés fonctionnelles identiques ou elles peuvent avoir une composition et des propriétés différentes.

Lorsque les couches 1 et 3 ont des propriétés fonctionnelles et des compositions identiques, elles peuvent différer par leurs quantités et leurs épaisseurs appliquées sur la couche 2.

Au moins une des couches 1 et 3 fait fonction de barrière, c'est-à-dire qu'elle est d'abord très peu perméable au passage du chlorhydrate d'alfuzosine contenu dans la couche 2 et au moins une des couches est caractérisée en ce qu'elle gonfle rapidement, c'est à dire qu'elle augmente rapidement de volume.

Une autre réalisation de la préparation pharmaceutique est caractérisée en ce que le comprimé à trois couches est formé par une première couche 1 telle que décrite ci-dessus c'est-à-dire qu'elle a pour seule fonction d'augmenter remarquablement de volume au contact des liquides aqueux, une deuxième couche 2 véhiculant une partie du chlorhydrate d'alfuzosine qui doit être libérée dans un intervalle de temps prédéterminé, et une troisième couche 3 dans laquelle est véhiculée une partie du chlorhydrate d'alfuzosine formulée de telle façon qu'elle puisse être immédiatement libérée au contact du suc gastrique.

La quantité de chlorhydrate d'alfuzosine véhiculée dans le comprimé est comprise entre 2,5 et 50 mg.

Les substances polymères qui sont utilisées dans les couches 1 et 3, et qui peuvent être aussi utilisées dans la couche 2, sont biocompatibles et ont des propriétés hydrophiles. Elles sont lentement solubles et/ou lentement gélifiables et/ou gonflent rapidement ou à une vitesse différente dans les liquides aqueux puis peuvent éventuellement s'éroder ; elles sont choisies dans le groupe suivant : l'hydroxyméthylcellulose, l'hydroxyéthylcellulose, l'hydroxypropylméthylcellulose ayant un poids moléculaire de 1000 à 4.000.000, l'hydroxypropylcellulose ayant un poids moléculaire de 2.000 à 2.000.000, les carboxyvinylpolymères, les chitosanes, les mannanes, les galactomannanes, les xanthanes, les carraghènanes, l'amylose, l'acide alginique, ses sels et ses dérivés, les pectines, les acrylates, les méthacrylates, les copolymères acryliques/méthacryliques, les polyanhydrides, les polyamminoacides, les poly(méthyl vinyl éthers/anhydride maléique), les alcools polyvinyliques, les glucanes, les scléroglucanes, la carboxyméthylcellulose et ses dérivés, l'éthylcellulose, la méthylcellulose et, en général, les dérivés cellulosiques hydrophiles.

La teneur en polymères hydrophiles peut aller de 5 à 90 % par rapport au poids total de la couche, mais de préférence de 10 à 85 % et plus particulièrement de 20 à 80%.

Afin de favoriser une augmentation rapide et remarquable du volume de la préparation pharmaceutique, lors de la préparation des couches 1 et 3, avec les polymères hydrophiles précédemment cités, on peut utiliser des produits et/ou des excipients hydrophiles capables de favoriser le mouillage des couches, en facilitant de cette façon l'interaction entre les composants de ladite couche et les fluides biologiques avec lesquels la couche entre en contact. Ces excipients hydrophiles sont choisis de préférence dans les groupes des excipients dits "superdésintégrants" comprenant la polyvinylpyrrolidone réticulée, l'hydroxypropylcellulose et l'hydroxypropylméthylcellulose ayant un poids moléculaire de 1000 à 1 000 000, la carboxyméthylcellulose sodique réticulée, le carboxyméthylamidon et ses sels, le copolymère divinylbenzène-méthacrylate de potassium.

Ces substances constituent de 1 à 50 % du poids de la couche et de préférence de 10 à 30 %.

Il est en outre possible d'utiliser aussi des substances tensioactives (anioniques, cationiques et non-ioniques) qui, en facilitant le mouillage, permettent une interaction plus rapide entre le milieu de dissolution (ou fluide gastrique) et le comprimé, ce qui permet un mouillage et un gonflement de la préparation pharmaceutique beaucoup plus rapide, de préférence de la couche dans laquelle cet élément modifiant l'hydratation est véhiculé. Dans le groupe de substances possédant ces propriétés on peut citer des produits tels que le laurylsulfate de sodium, le ricinoléate de sodium, le tétradécylsulfate de sodium, le dioctylsulfosuccinate de sodium, le cétomacrogol, le poloxamère, le monostéarate de glycérol, les polysorbates, le sorbitanemonolaurate, les lécithines ou tout autre tensio-actif pharmaceutiquement acceptable.

En outre on peut utiliser d'autres éléments modifiant l'hydratation choisis parmi le groupe de substances suivantes:
- les diluants hydrophiles tels que le mannitol, le lactose, des amidons d'origines différentes, le sorbitol, le xylitol, la cellulose microcristalline et/ou en général des substances qui favorisent la pénétration de l'eau ou des fluides aqueux dans la préparation pharmaceutique,
- les diluants hydrophobes tels que le monostéarate de glycérol, les palmitates, les huiles végétales hydrogénées ou non telles que l'huile de ricin hydrogénée, des cires, des glycérides mono, bi ou trisubstitués, pour ralentir la pénétration de l'eau ou des fluides aqueux dans la préparation pharmaceutique.

La réalisation technique des comprimés peut amener à introduire :
- des agents lubrifiants tels que le stéarate de magnésium, l'acide stéarique, le monostéarate de glycérol, les polyoxyethylèneglycols ayant un poids moléculaire de 400 à 7.000.000, l'huile de ricin hydrogénée, le béhénate de glycérol, les glycérides mono, bi ou trisubstitués,
- des agents d'écoulement, tels que la silice colloïdale ou toute autre silice,
- et des liants, tampons, absorbants, ainsi que tout autre additif pharmaceutiquement acceptable.

Les comprimés de l'invention peuvent être produits de la manière suivante : on mélange des poudres et/ou des granulés en utilisant les technologies de production actuelles donc avec un procédé de production qui peut être immédiatement transféré sur le plan industriel.

Le comprimé pharmaceutique à deux ou trois couches est obtenu selon des procédés de compression très utilisés et connus par l'homme du métier.

Par exemple on peut produire les comprimés en utilisant des presses rotatives capables de produire des comprimés "multi-couches".

Normalement, la force de compression de travail varie de 7 à 50 KN (ou kilo newtons), et l'on obtient, selon les procédés qui seront décrits d'une façon plus détaillée dans les exemples, des comprimés à deux ou trois couches ayant une forme cylindrique, lenticulaire, sphéroïdale, ovoïdale, qui permettent une administration et une déglutition faciles.

Selon la quantité de substance active qui est véhiculée chaque couche du comprimé peut avoir une épaisseur différente allant de 0,2 à 8 mm, mais de préférence de 1 mm à 4 mm.

A la préparation pharmaceutique on peut en outre appliquer un enrobage en matériaux polymèriques ayant pour but une simple protection ou bien un ralentissement au début de la libération de la substance active véhiculée dans la préparation pharmaceutique. L'enrobage pourra être soluble en solution acide ou bien perméable de telle façon à permettre l'activation du comprimé (libération de la substance active) seulement après un intervalle de temps prédéterminé.

Selon un autre de mode de réalisation de l'invention, on peut appliquer un enrobage soluble contenant du chlorhydrate d'alfuzosine de telle façon à permettre une libération immédiate d'une partie de la substance active au contact des sucs gastriques.

L'enrobage peut être appliqué par des méthodes classiques connues de l'homme du métier à l'aide de solutions organiques ou aqueuses.

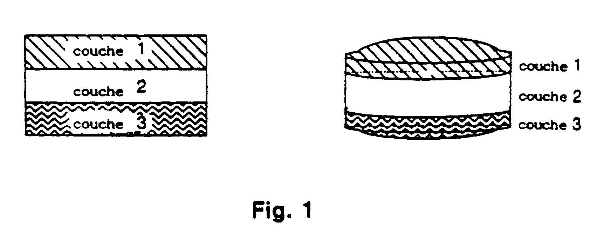

La figure 1 présente un mode de réalisation de l'invention comprenant un comprimé à trois couches comme décrit ci-dessus.

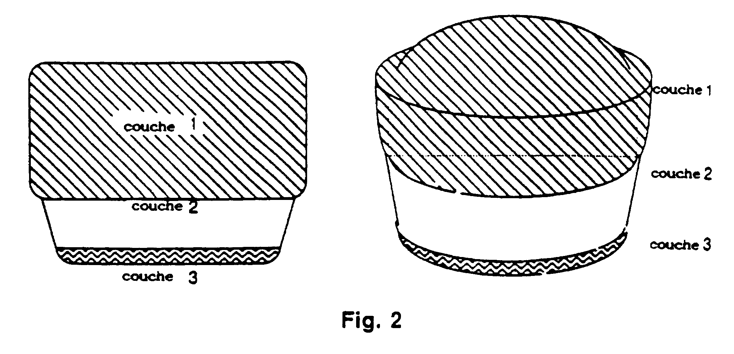

Le comprimé au contact du suc gastrique et/ou des fluides du tractus gastro-intestinal augmente rapidement de volume en prenant la structure montrée dans la figure 2.

Cette augmentation de volume peut être déterminée et limitée à une seule couche ou à plusieurs couches du comprimé ; cette augmentation de volume, ainsi que la vitesse avec laquelle ce phénomène a lieu, peuvent être suivies et évaluées avec précision par des mesures directes ou par un vidéomicroscope accouplé à un ordinateur. La mesure est élaborée par un programme spécial d'analyse vidéo.

Le comprimé est caractérisé par le fait que le volume d'au moins une des couches augmente, au bout de 2 heures, de 1,5 fois et de préférence d'au moins 3 fois par rapport au volume initial.

Par cette méthode il est possible d'effectuer l'étude du comportement "in vitro" des diverses préparations (décrites dans les exemples de la demande) et donc de concevoir des préparations pharmaceutiques capables d'être conformes aux qualités morphologiques demandées, ainsi que d'optimiser la préparation de chacune des dites couches de façon à obtenir le comportement morphologique répondant au but demandé. Ce genre d'analyse permet donc de modéliser le comportement "in vivo" de la préparation pharmaceutique au contact des fluides biologiques. Il est en outre possible de programmer dans un intervalle de temps déterminé, la libération de la substance active véhiculée dans la préparation pharmaceutique.

Les compositions pharmaceutiques de la présente invention peuvent se présenter sous forme de comprimés ou de petits comprimés ou de gélules comprenant de petits comprimés.

On peut également associer dans une même composition pharmaceutique au moins deux petits comprimés. Ils peuvent être conditionnés dans une enveloppe commune, par exemple dans une capsule ou dans une gélule.

Quand la composition pharmaceutique est constituée de petits comprimés, chacun de ceux-ci peut avoir une composition différente ou identique.

Les exemples qui suivent ont pour but d'illustrer l'invention.

### Exemple 1 :

### Préparation d'une série de comprimés (5000) à base de chlorhydrate d'alfuzosine.

### 1 A : Préparation du granulé contenant la substance active

On prépare un granulé, selon le procédé décrit ci-dessous, utilisé pour la préparation de la couche 2 de la figure 1 contenant 10,0 mg de chlorhydrate d'alfuzosine et ayant la composition unitaire suivante :

| | |
|---|---|
| Chlorhydrate d'alfuzosine | 10,00 mg |
| Mannitol | 10,00 mg |
| Hydroxypropylméthylcellulose USP 2208 | 10,00 mg |
| Polyvinylpyrrolidone | 3,20 mg |
| Cellulose microcristalline | 65,00 mg |
| Stéarate de magnésium | 1,00 mg |
| Silice colloïdale | 1,25 mg |
| Total | 100,45 mg |

Le procédé de fabrication consiste à préparer un granulé en mélangeant les quantités de substance active nécessaire, de mannitol, de cellulose microcristalline et d'hydroxypropylméthylcellulose. Le mélange de poudres homogène est mouillé de façon homogène avec une solution alcoolique à base de polyvinylpyrrolidone à 10 % m/v puis est séché jusqu'à un pourcentage d'humidité résiduelle prédéterminé dans un lit d'air fluidisé à 40-45°C. Le granulé séché est calibré et placé dans un mélangeur à poudres avec du stéarate de magnésium et de la silice colloïdale puis mélangé jusqu'à homogénéité.

### 1 B : Préparation du granulé constituant la couche 1 qui gonfle

On prépare une quantité de granulé nécessaire pour obtenir 5.000 couches qui gonflent, couche 1 de la figure 1, chaque couche ayant la composition suivante en pourcentage :

| | |
|---|---|
| Hydroxypropylméthylcellulose | 79,75 % |
| Huile de ricin hydrogénée | 13,50 % |
| Oxyde de fer jaune | 0,25 % |
| Ethylcellulose | 5,00 % |
| Stéarate de magnésium | 1,00 % |
| Gel de silice | 0,50 % |
| Total | 100,00 % |

Le procédé de fabrication consiste en la préparation d'un granulé obtenu en mélangeant les quantités nécessaires d'hydroxypropylméthylcellulose, huile de ricin hydrogénée et oxyde de fer ; le mélange de poudres homogène est mouillé avec une solution alcoolique à base d'éthylcellulose à 10 % m/v et la masse mouillée d'une façon homogène est séchée dans un lit d'air fluidisé à 40-45°C. Le granulé, séché jusqu'à un pourcentage d'humidité prédéterminé, est calibré et placé dans un mélangeur à poudres avec du stéarate de magnésium et de la silice colloïdale et il est mélangé jusqu'à homogénéité.

### 1 C : Préparation du granulé constituant la troisième couche 3 qui fait barrière

On prépare une quantité de granulé nécessaire pour obtenir 5.000 couches barrières, couche 3 de la figure 1, chaque couche ayant la composition suivante en pourcentage :

| | |
|---|---|
| Hydroxypropylméthylcellulose | 76,00 % |
| Huile de ricin hydrogénée | 18,60 % |
| Polyvinylpyrrolidone | 3,15 % |
| Oxyde de fer jaune | 0,10 % |
| Stéarate de magnésium | 0,70 % |
| Silice colloïdale | 1,45 % |
| Total | 100,00 % |

Le procédé de fabrication consiste à mélanger les quantités nécessaires d'hydroxypropylméthylcellulose, huile de ricin hydrogénée et oxyde de fer jaune ; le mélange de poudres homogène est mouillé avec une solution à base de polyvinylpyrrolidone à 10 % m/v dans l'éthanol et la masse mouillée est séchée dans un lit d'air fluidisé à 40-45°C. Le granulé séché jusqu'à un pourcentage d'humidité résiduelle prédéterminé, est calibré et placé dans un mélangeur à poudres avec du stéarate de magnésium et de la silice colloïdale et mélangé jusqu'à homogénéité.

### 1 D : Préparation des comprimés à trois couches (par compression)

Les granulés obtenus sont chargés dans les trois trémies d'alimentation d'une presse multi-couches rotative capable de produire des comprimés à trois couches. Dans la première trémie on charge le granulé décrit au point 1 B ; dans la deuxième trémie on charge le granulé selon la description du point 1 A ; tandis que dans la troisième trémie on charge le granulé selon la description du point 1 C; les granulés 1 B et 1 C peuvent être inversés dans les trémies.

La presse multi-couches est équipée de poinçons circulaires plats avec chanfrein ayant un diamètre de 8 mm. La machine est ajustée pour produire des comprimés à trois couches constitués par une première quantité de 100 mg de couche 1 pour une épaisseur de 1,7 mm environ, une deuxième quantité de 100,45 mg de granulé contenant la substance active (équivalent à 10,0 mg de chlorhydrate d'alfuzosine) et une troisième quantité de 150 mg de couché 3 pour une épaisseur de 3,3 mm environ. En opérant selon la description précédente, on produit des comprimés à trois couches ayant un poids moyen de 350,45 mg contenant 10,0 mg de chlorhydrate d'alfuzosine.

### 1 E :Test de dissolution

Afin d'évaluer les propriétés de libération des comprimés complets, on utilise l'appareil à palette (décrit dans l'USP XXIII) opérant à 100 t.p.m. et en utilisant comme liquide de dissolution une solution de HC1 0,01 M à 37°C. La libération de la substance active est suivie par détermination spectrophotométrique U.V. à 330 nm en utilisant un système d'échantillonnage et de lecture automatique.
Les résultats des tests effectués sont rapportés dans le tableau 1.

**TABLEAU 1**

| Temps (heures) | % libéré |
|---|---|
| 1 | 16,0 |
| 2 | 25,0 |
| 3 | 32,0 |
| 4 | 37,0 |
| 6 | 48,0 |
| 8 | 57,0 |
| 10 | 66,0 |
| 12 | 74,0 |
| 16 | 88,0 |
| 20 | 95,0 |
| 24 | 98,0 |

On obtient une libération contrôlée de la substance active en 20 heures environ.

### 1 F : Test de gonflement

Le test est conduit dans les mêmes conditions expérimentales que le test de dissolution. Les comprimés sont prélevés du milieu de dissolution à intervalles de temps réguliers et leur volume et les dimensions des différentes couches sont mesurés avec un vidéomicroscope accouplé à un système d'analyse de l'image. Les résultats des tests effectués sont rapportés dans le tableau 2.

**TABLEAU 2**

| Temps (heures) gonflement | Volume (couche 2 + couche 3) (%) | volume couche 1 (%) |
|---|---|---|
| 0 | 100,0 | 100,0 |
| 0,5 | 142,0 | 211,1 |
| 1 | 152,7 | 271,0 |
| 1,5 | 175,2 | 302,6 |
| 2 | 161,8 | 399,5 |
| 3 | 182,7 | 483,7 |
| 4 | 196,0 | 534,0 |
| 5 | 199,4 | 609,8 |
| 6 | 195,7 | 727,9 |
| 7 | 166,8 | 809,9 |
| 8 | 138,9 | 851,0 |
| 10 | 139,9 | 937,5 |

On peut remarquer que dans les comprimés la couche 1 augmente de volume de façon remarquable, jusqu'à 9 fois son volume initial. Ce phénomène est très évident s'il est rapporté à l'augmentation de volume des deux autres couches, couche 2 et couche 3, qui gonflent cumulativement jusqu'à 2 fois environ. En outre la couche 1 augmente de volume à une vitesse remarquablement supérieure à celle des deux autres couches.

### Exemple 2

Préparation d'une série de comprimés (10.000) comme rapporté dans les figures 1 et 2, contenant comme substance active du chlorhydrate d'alfuzosine.

### 2 A : Préparation du granulé contenant la substance active

On prépare un granulé, selon le procédé décrit dans l'exemple 1 A, qui est utilisé dans la préparation de la couche 2 de la figure 1 contenant 7,5 mg de chlorhydrate d'alfuzosine, et ayant la composition unitaire suivante :

| | |
|---|---|
| Chlorhydrate d'alfuzosine | 7,50 mg |
| Mannitol | 10,00 mg |
| Hydroxypropylméthylcellulose | 10,00 mg |
| Polyvinylpyrrolidone | 3,20 mg |
| Cellulose microcristalline | 65,00 mg |
| Stéarate de magnésium | 1,00 mg |
| Silice colloïdale | 1,25 mg |
| Total | 97,95 mg |

### 2 B : Préparation du granulé constituant la première couche 1 qui gonfle

On prépare, selon le procédé décrit dans l'exemple 1 B, une quantité de granulé nécessaire pour obtenir 10.000 couches qui gonflent, couche 1 de la figure 1, ayant chacune la composition unitaire suivante en pourcentage :

| | |
|---|---|
| Hydroxypropylméthylcellulose | 79,75 % |
| Huile de ricin hydrogénée | 13,50 % |
| Ethylcellulose | 5,00 % |
| Oxyde de fer | 0,25 % |
| Stéarate de magnésium | 1,00 % |
| Silice colloïdale | 0,50 % |
| Total | 100,00 % |

### 2 C:Préparation du granulé constituant la troisième couche 3

On prépare, selon le procédé décrit dans l'exemple 1 C, une quantité de granulé nécessaire pour obtenir 10.000 couches barrières, couche 3 de la figure 1 ayant chacune la composition unitaire suivante en pourcentage :

| | |
|---|---|
| Hydroxypropylméthylcellulose | 76,00 % |
| Huile de ricin hydrogénée | 18,60 % |
| Polyvinylpyrrolidone | 3,15 % |
| Oxyde de fer jaune | 0,10 % |
| Stéarate de magnésium | 1,45 % |
| Silice colloïdale | 0,70 % |
| Total | 100,00 % |

### 2 D: Préparation des comprimés à trois couches (par compression)

Les granulés obtenus selon les exemples 2 A, 2 B et 2 C sont chargés dans les trois trémies d'alimentation d'une presse rotative avec respectivement les quantités de 100 mg de granulé pour la couche 1 pour une épaisseur de 1,75 mm, de 97,95 mg de granulé contenant la substance active (correspondant à 7,5 mg de chlorhydrate d'alfuzosine) pour la couche 2 et de 150 mg pour la couche 3 pour une épaisseur de 3,3 mm. En opérant de la façon décrite ci-dessus, on obtient des comprimés à trois couches ayant un poids moyen de 347,95 mg contenant 7,5 mg de substance active.

### 2 E: Test de dissolution

Les tests de dissolution sont effectués selon le procédé décrit dans l'exemple 1 E.
Les résultats sont rapportés dans le tableau 3.

**TABLEAU 3**

| Temps (heures) | % libéré |
|---|---|
| 1 | 15,1 |
| 2 | 24,4 |
| 4 | 37,7 |
| 6 | 48,0 |
| 8 | 57,6 |
| 10 | 66,0 |
| 12 | 74,2 |
| 14 | 82,2 |
| 16 | 89,1 |
| 18 | 94,8 |
| 20 | 98,6 |

On peut remarquer que la libération contrôlée de la substance active se fait en 20 heures environ.

### 2 F: Test de gonflement

Les tests de gonflement sont effectués selon le procédé décrit dans l'exemple 1 F. Les résultats sont rapportés dans le tableau 4.

**TABLEAU 4**

| Temps (heures) | Volume (couche 2 + couche 3) (%) | Volume couche 1 (%) |
|---|---|---|
| 0 | 100,0 | 100,0 |
| 0,5 | 137,6 | 233,2 |
| 1 | 142,3 | 305,1 |
| 1,5 | 150,4 | 338,5 |
| 2 | 142,3 | 412,4 |
| 3 | 167,1 | 435,2 |
| 4 | 139,2 | 526,5 |
| 6 | 132,0 | 665,0 |
| 8 | 129,9 | 715,1 |

On peut remarquer que dans les comprimés préparés le volume de la couche 1 augmente d'une façon remarquable jusqu'à 7 fois le volume initial ; la couche 2 et la couche 3 augmentent jusqu'à 1 fois et demi. En outre la couche 1 augmente de volume à une vitesse très supérieure à celle des deux autres couches.

### Exemple 3 : Préparation d'une série de comprimés (10.000) contenant comme substance active le chlorhydrate d'alfuzosine

### 3 A: Préparation du granulé contenant la substance active.

On prépare, selon le procédé décrit dans l'exemple 1 A, un granulé utilisé dans la préparation de la couche 2, contenant 10,0 mg de chlorhydrate d'alfuzosine et ayant la composition unitaire suivante:

| | |
|---|---|
| Chlorhydrate d'alfuzosine | 10,00 mg |
| Mannitol | 10,00 mg |
| Hydroxypropylméthylcellulose | 10,00 mg |
| Polyvinylpyrrolidone | 3,20 mg |
| Cellulose microcristalline | 65,00 mg |
| Stéarate de magnésium | 1,00 mg |
| Silice colloïdale | 1,25 mg |
| Total | 100,45 mg |

### 3 B: Préparation du granulé constituant la première couche 1 qui gonfle

On prépare, selon le procédé décrit une quantité de granulé nécessaire pour obtenir 10.000 couches qui gonflent, couche 1 de la figure 1, ayant chacune la composition suivante en pourcentage:

| | |
|---|---|
| Hydroxypropylméthylcellulose | 75,00 % |
| Béhénate de glycérol | 13,40 % |
| Polyvinylpyrrolidone | 5,00 % |
| Oxyde de fer | 0,10 % |
| Polyvinylpyrrolidone | 5,00 % |
| Stéarate de magnésium | 1,00 % |
| Silice colloïdale | 0,50 % |
| Total | 100,00 % |

### 3 C: Préparation du granulé constituant la troisième couche 3

On prépare, selon le procédé décrit dans l'exemple 1 C, une quantité de granulé nécessaire pour obtenir 10.000 couches, couche 3 de la figure 1, ayant chacune la composition suivante en pourcentage :

| | |
|---|---|
| Hydroxypropylméthylcellulose | 76,00 % |
| Huile de ricin hydrogénée | 18,60 % |
| Polyvinylpyrrolidone | 3,15 % |
| Oxyde de fer jaune | 0,10 % |
| Stéarate de magnésium | 1,45 % |
| Silice colloïdale | 0,70 % |
| Total | 100,0 % |

### 3 D: Préparation des comprimés à trois couches (par compression)

Les granulés obtenus comme décrit dans les exemples 3 A, 3 B et 3 C avec les quantités respectives de 100 mg de granulé pour la couche 1, de 100,45 mg de granulé contenant la substance active pour la couche 2 et de 150 mg pour la couche 3. En opérant de la façon décrite ci-dessus, on obtient des comprimés à trois couches ayant un poids moyen de 350,45 mg contenant 10,0 mg de substance active.

### 3 E: Test de dissolution

Les tests de dissolution sont effectués selon le procédé décrit dans l'exemple 1 E.

Les résultats des tests effectués sont rapportés dans le tableau 5.

**TABLEAU 5**

| Temps (heures) | % libéré |
|---|---|
| 1 | 19,0 |
| 2 | 27,8 |
| 4 | 41,7 |
| 6 | 53,4 |
| 8 | 64,7 |
| 10 | 75,6 |
| 12 | 84,6 |
| 14 | 90,9 |
| 16 | 95,1 |
| 18 | 97,8 |
| 20 | 99,4 |

La libération contrôlée de la substance active se fait en 18 heures environ.

### 3 F: Test de gonflement

Les tests de gonflement sont effectués selon le procédé décrit dans l'exemple 1 F.
Les résultats des tests effectués sont rapportés dans le tableau 6.

**TABLEAU 6**

| Temps (heures) | Volume (couche 2 + couche 3) (%) | Volume couche 1 (%) |
|---|---|---|
| 0 | 100,0 | 100,0 |
| 0,5 | 124,0 | 231,8 |
| 1 | 130,5 | 297,0 |
| 2 | 108,5 | 387,0 |
| 3 | 115,2 | 448,8 |
| 4 | 131,3 | 517,2 |
| 5 | 124,7 | 554,5 |
| 6 | 137,0 | 601,1 |
| 8 | 106,6 | 740,5 |

On peut remarquer que dans les comprimés préparés le volume de la couche 1 qui gonfle augmente d'une façon remarquable, jusqu'à 7 fois son volume initial ; la couche 2 et la couche 3 augmentent seulement de 30-40 % par rapport au volume initial. En outre la couche qui gonfle augmente de volume à une vitesse très supérieure par rapport à celle des deux autres couches.

### Exemple 4 :

### Préparation d'une série de comprimés (5000) à base de chlorhydrate d'alfuzosine.

### 4 A : Préparation du granulé contenant la substance active

On prépare un granulé, selon le procédé décrit ci-dessous, utilisé pour la préparation de la couche 2 de la figure 1 contenant 10,0 mg de chlorhydrate d'alfuzosine et ayant la composition unitaire suivante :

| | |
|---|---|
| Chlorhydrate d'alfuzosine | 10,00 mg |
| Lactose | 60,30 mg |
| Hydroxypropylméthylcellulose USP 2208 | 25,00 mg |
| Polyvinylpyrrolidone | 3,20 mg |
| Stéarate de magnésium | 1,00 mg |
| Silice colloïdale | 0,50 mg |
| Total | 100,00 mg |

Le procédé de fabrication consiste à préparer un granulé en mélangeant les quantités de substance active nécessaire, de lactose, de polyvinylpyrrolidone et d'hydroxypropylméthylcellulose. Le mélange de poudres homogène est mouillé de façon homogène avec de l'eau purifiée puis est séché jusqu'à un pourcentage d'humidité résiduelle prédéterminé dans un lit d'air fluidisé à 40-45°C. Le granulé séché est calibré et placé dans un mélangeur à poudres avec du stéarate de magnésium et de la silice colloïdale puis mélangé jusqu'à homogénéité.

### 4 B : Préparation du granulé constituant la couche 1 et 3 qui gonflent et font barrière

On prépare une quantité de granulé nécessaire pour obtenir 10000 couches qui gonflent et font barrière, couche 1 et 3 de la figure 1, chaque couche ayant la composition suivante en pourcentage :

| | |
|---|---|
| Hydroxypropylméthylcellulose USP 2208 | 40,00 % |
| Lactose | 39,75 % |
| Béhénate de glycérol | 13,50 % |
| Oxyde de fer jaune | 0,25 % |
| Polyvinylpyrrolidone | 5,00 % |
| Stéarate de magnésium | 1,00 % |
| Silice colloïdale | 0,50 % |
| Total | 100,00 % |

Le procédé de fabrication consiste en la préparation d'un granulé obtenu en mélangeant les quantités nécessaires d'hydroxypropylméthylcellulose, de lactose, de béhénate de glycérol, de polyvinylpyrrolidone et d'oxyde de fer ; le mélange de poudres homogène est mouillé avec de l'eau purifiée. La masse, mouillée d'une façon homogène, est séchée dans un lit d'air fluidisé à 40-45°C. Le granulé, séché jusqu'à un pourcentage d'humidité prédéterminé, est calibré et placé dans un mélangeur à poudres avec du stéarate de magnésium et de la silice colloïdale et il est mélangé jusqu'à homogénéité.

### 4 C : Préparation des comprimés à trois couches (par compression)

Les granulés obtenus sont chargés dans les trois trémies d'alimentation d'une presse multi-couches rotative capable de produire des comprimés à trois couches. Dans la première et la troisième trémie on charge le granulé décrit au point 4 B ; dans la deuxième trémie on charge le granulé selon la description du point 4 A.
La presse multi-couches est équipée de poinçons circulaires plats avec chanfrein ayant un diamètre de 8 mm. La machine est ajustée pour produire des comprimés à trois couches constitués par une première quantité de 100 mg de couche 1 ou 3 pour une épaisseur de 1,7 mm environ, une deuxième quantité de 100 mg de granulé contenant la substance active et une troisième quantité de 100 mg de couche 1 ou 3 pour une épaisseur de 1,7 mm environ. En opérant selon la description précédente, on produit des comprimés à trois couches ayant un poids moyen de 300 mg contenant 10,0 mg de chlorhydrate d'alfuzosine.

### Exemple 5

### Préparation d'une série de comprimés (5000) à base de chlorhydrate d'alfuzosine.

### 5 A : Préparation du granulé contenant la substance active

On prépare un granulé, selon le procédé décrit dans l'exemple 4 A, qui est utilisé dans la préparation de la couche 2 de la figure 1 contenant 15 mg de chlorhydrate d'alfuzosine, et ayant la composition unitaire suivante :

| | |
|---|---|
| Chlorhydrate d'alfuzosine | 15,00 mg |
| Lactose | 55,30 mg |
| Hydroxypropylméthylcellulose USP 2208 | 25,00 mg |
| Polyvinylpyrrolidone | 3,20 mg |
| Stéarate de magnésium | 1,00 mg |
| Silice colloïdale | 0,50 mg |
| Total | 100,00 mg |

### 5 B : Préparation du granulé constituant les couches 1 et 3 qui gonflent et font barrière

On prépare une quantité de granulé nécessaire pour obtenir 10000 couches qui gonflent et font barrière, couches 1 et 3 de la figure 1, chaque couche correspond à la composition et au procédé de fabrication décrit dans l'exemple 4 B.

### 5 C : Préparation des comprimés à trois couches (par compression)

En opérant de la façon décrite ci-dessus (exemple 4C), on obtient, avec les granulés décrits dans les exemples 5 A et 5B, des comprimés à trois couches contenant 15,0 mg de substance active.

### Exemple 6

### Préparation d'une série de comprimés (5000) à base de chlorhydrate d'alfuzosine.

### 6 A : Préparation du granulé contenant la substance active

On prépare un granulé, selon le procédé décrit ci-dessous, utilisé dans la préparation de la couche 2 de la figure 1 contenant 10 mg de chlorhydrate d'alfuzosine, et ayant la composition unitaire suivante :

| | |
|---|---|
| Chlorhydrate d'alfuzosine | 10,00 mg |
| Cellulose microcristalline | 33,80 mg |
| Mannitol | 10,00 mg |
| Hydroxypropylméthylcellulose USP2208 | 40,00 mg |
| Polyvinylpyrrolidone | 5,00 mg |
| Stéarate de magnésium | 1,00 mg |
| Silice colloïdale | 0,20 mg |
| Total | 100,00 mg |

Le procédé de fabrication consiste à préparer un granulé en mélangeant les quantités de substance active nécessaire, de cellulose, de polyvinylpyrrolidone, de mannitol et d'hydroxypropylméthylcellulose. Le mélange de poudres homogène est mouillé de façon homogène avec de l'eau purifiée puis est séché jusqu'à un pourcentage d'humidité résiduelle prédéterminé dans un lit d'aire fluidisé à 40-50 °C. Le granulé séché est calibré et placé dans un mélangeur à poudres avec du stéarate de magnésium et de la silice colloïdale jusqu'à homogénéité.

### 6 B : Préparation du granulé constituant les couches 1 et 3 qui gonflent et font barrière

On prépare une quantité de granulé nécessaire pour obtenir 10000 couches qui gonflent et font barrière, couches 1 et 3 de la figure 1, chaque couche correspond à la composition ayant la composition suivante en pourcentage :

| | |
|---|---|
| Hydroxypropylméthylcellulose USP 2208 | 45,00 % |
| Lactose | 28,60 % |
| Cellulose microcristalline | 20,00 % |
| Oxyde de fer jaune | 0,20 % |
| Polyvinylpyrrolidone | 5,00 % |
| Stéarate de magnésium | 1,00 % |
| Silice colloïdale | 0,20 % |
| Total | 100,00 % |

Le procédé de fabrication est identique à celui de l'exemple 4B, la cellulose microcristalline étant ajoutée à la place du béhénate de glycérol.

### 6C : Préparation des comprimés à trois couches (par compression)

En opérant de la façon décrite ci-dessus (exemple 4C), on obtient, avec 100 mg, pour chacune des couches, de granulés décrits dans les exemples 6 A et 6B, des comprimés à trois couches contenant 10,0 mg de substance active, les couches 1 et 3 ayant pour épaisseur environ 1,8 mm.

### Exemple 7

### Préparation d'une série de comprimés (5000) à base de chlorhydrate d'alfuzosine.

### 7 A : Préparation du granulé contenant la substance active

On prépare un granulé, selon le procédé décrit ci-dessous, utilisé dans la préparation de la couche 2 de la figure 1 contenant 15 mg de chlorhydrate d'alfuzosine, et ayant la composition unitaire suivante :

| | |
|---|---|
| Chlorhydrate d'alfuzosine | 15,00 mg |
| Cellulose microcristalline | 28,80 mg |
| Mannitol | 10,0 mg |
| Hydroxypropylméthylcellulose USP2208 | 40,00 mg |
| Polyvinylpyrrolidone | 5,00 mg |
| Stéarate de magnésium | 1,00 mg |
| Silice colloïdale | 0,20 mg |
| Total | 100,00 mg |

Le procédé de fabrication est identique à celui de l'exemple 6A.

### 7 B : Préparation du granulé constituant les couches 1 et 3 qui gonflent et font barrière

On prépare une quantité de granulé nécessaire pour obtenir 10000 couches qui gonflent et font barrière, couches 1 et 3 de la figure 1, chaque couche correspond à la composition et au procédé de fabrication décrit dans l'exemple 6B.

### 7C : Préparation des comprimés à trois couches (par compression)

En opérant de la façon décrite ci-dessus (exemple 6C), on obtient, avec les granulés décrits dans les exemples 7 A et 7B, des comprimés à trois couches contenant 15,0 mg de substance active.

### Exemple 8

### Préparation d'une série de comprimés (5000) à base de chlorhydrate d'alfuzosine.

### 8 A : Préparation du granulé contenant la substance active

On prépare un granulé, utilisé dans la préparation de la couche 2 de la figure 1, contenant 10 mg de chlorhydrate d'alfuzosine, avec une composition identique à celle décrite dans l'exemple 6A et selon le même procédé.

### 8 B : Préparation du granulé constituant les couches 1 et 3 qui gonflent et font barrière

On prépare une quantité de granulé nécessaire pour obtenir 10000 couches qui gonflent et font barrière, couches 1 et 3 de la figure 1, chaque couche correspond à la composition ayant la composition suivante en pourcentage :

| | |
|---|---|
| Hydroxypropylméthylcellulose USP 2208 | 35,00 % |
| Lactose | 34,50 % |
| Cellulose microcristalline | 23,90 % |
| Oxyde de fer jaune | 0,40 % |
| Polyvinylpyrrolidone | 5,00 % |
| Stéarate de magnésium | 1,00 % |
| Silice colloïdale | 0,20 % |
| Total | 100,00 % |

Le procédé de fabrication est identique à celui de l'exemple 6B.

### 8 C : Préparation des comprimés à trois couches (par compression)

Les granulés obtenus sont chargés dans les trois trémies d'alimentation d'une presse multi-couches rotative capable de produire des comprimés à trois couches. Dans la première et la troisième trémie on charge le granulé décrit au point 8 B; dans la deuxième trémie on charge le granulé selon la description du point 8 A.

La presse multi-couches est équipée de poinçons circulaires plats avec chanfrein ayant un diamètre de 8 mm. La machine est ajustée pour produire des comprimés à trois couches constitués pour les couches externes de 100 mg et 150 mg du granulé décrit au point 8B et correspondant respectivement à une épaisseur de 1,7 mm environ pour l'une et 2,7 mm pour l'autre. La couche interne est composée de 100 mg de granulé contenant la substance active (équivalent à 10,0 mg de chlorhydrate d'alfuzosine) . En opérant selon la description précédente au point 7C, on produit des comprimés à trois couches ayant un poids moyen de 350 mg contenant 10,0 mg de chlorhydrate d'alfuzosine.

### Exemple 9

### Préparation d'une série de comprimés (5000) à base de chlorhydrate d'alfuzosine.

### 9 A : Préparation du granulé contenant la substance active

On prépare un granulé, utilisé dans la préparation de la couche 2 de la figure 1, contenant 15 mg de chlorhydrate d'alfuzosine, avec une composition identique à celle décrite dans l'exemple 7A et selon le même procédé.

### 9 B : Préparation du granulé constituant les couches 1 et 3 qui gonflent et font barrière

On prépare une quantité de granulé nécessaire pour obtenir 10000 couches qui gonflent et font barrière, couches 1 et 3 de la figure 1, chaque couche correspond à la composition et au procédé de fabrication décrit dans l'exemple 8B.

### 9 C : Préparation des comprimés à trois couches (par compression)

En opérant de la façon décrite ci-dessus (exemple 8C), on obtient, avec 100 mg de granulé décrit dans l'exemple 9 A et pour les couches externes 100 et 150 mg de granulé décrit au point 9 B correspondant respectivement à une épaisseur d'environ 1,8 mm pour l'une et 2,7 mm pour l'autre, des comprimés à trois couches contenant 15,0 mg de substance active et un poids moyen de 350 mg.

### Exemple 10

### Préparation d'une série de comprimés (5000) à base de chlorhydrate d'alfuzosine.

### 10 A : Préparation du granulé contenant la substance active

On prépare un granulé, utilisé dans la préparation de la couche 2 de la figure 1, contenant 7,5 mg de chlorhydrate d'alfuzosine, avec une composition identique à celle décrite dans l'exemple 2A et selon le même procédé.

### 10 B : Préparation du granulé constituant les couches 1 et 3 qui gonflent et font barrière

On prépare une quantité de granulé nécessaire pour obtenir 10000 couches qui gonflent et font barrière, couches 1 et 3 de la figure 1, chaque couche correspond à la composition et au procédé de fabrication décrit dans l'exemple 4 B.

### 10 C : Préparation des comprimés à trois couches (par compression)

En opérant de la façon décrite ci-dessus (exemple 8C), on obtient, avec 100 mg de granulé décrit dans l'exemple 10 A et pour les couches externes 100 et 150 mg de granulé décrit au point 10 B correspondant respectivement à une épaisseur d'environ 1,8 mm pour l'une et 2,7 mm pour l'autre, des comprimés à trois couches contenant 7,5 mg de substance active et un poids moyen de 350 mg.

### Exemple 11

### Préparation d'une série de comprimés (5000) à base de chlorhydrate d'alfuzosine.

### 11 A : Préparation du granulé contenant la substance active

On prépare un granulé, contenant 10 mg de chlorhydrate d'alfuzosine, avec une composition identique à celle décrite dans l'exemple 4A et selon le même procédé.

### 11 B : Préparation du granulé constituant la couche 1 qui gonfle

On prépare une quantité de granulé nécessaire pour obtenir 10000 couches qui gonflent. Chaque couche correspond à la composition et au procédé de fabrication décrit dans l'exemple 4 B.

### 11 C : Préparation des comprimés à deux couches (par compression)

En opérant de la façon décrite ci-dessus (exemple 8C), les granulés obtenus, 100 mg de granulé décrit dans l'exemple 10 A et pour la couche externe 150 mg de granulé décrit au point 10 B, sont chargés dans deux trémies d'alimentation d'une presse multi-couches rotative capable de produire des comprimés à deux couches.

## Revendications

1. Comprimé pharmaceutique destiné à la voie orale, pour la libération contrôlée de chlorhydrate d'alfuzosine au niveau des segments proximaux du tractus gastro-intestinal, comprimé caractérisé en ce qu'il est constitué par :
a) une première couche 1 ayant la propriété de gonfler remarquablement et rapidement au contact avec les liquides biologiques aqueux, ladite couche étant produite par compression d'un mélange ou d'un granulé comprenant des polymères hydrophiles constituant de 5.0 à 90 % et de préférence de 10 à 85 % du poids de la couche,
b) une deuxième couche 2 adjacente ou superposée à la première couche, dans laquelle le chlorhydrate d'alfuzosine est véhiculé, cette couche étant formulée avec des polymères hydrophiles et avec d'autres substances auxiliaires, afin de donner à la préparation des propriétés de compressibilité convenables et pour permettre la libération de chlorhydrate d'alfuzosine dans un intervalle de temps prédéterminé,
c) et éventuellement une troisième couche 3 obtenue par compression et appliquée sur la couche 2, généralement constituée en particulier par des polymères hydrophiles qui gélifient et/ou gonflent puis peuvent éventuellement s'éroder et ayant fonction de barrière modulant la libération du chlorhydrate d'alfuzosine de la couche 2, la couche 3 étant d'abord très peu perméable au passage de la substance active.

2. Comprimé selon la revendication 1, caractérisé en ce que pour favoriser un gonflement rapide, au moins une des couches est constituée de produits et/ou d'excipients hydrophiles.

3. Comprimé selon la revendication 2, caractérisé en ce que les excipients hydrophiles appartiennent au groupe des "superdésintégrants" comprenant la polyvinylpyrrolidone réticulée, l'hydroxypropylcellulose et l'hydroxypropylméthylcellulose ayant un poids moléculaire de 1000 à 1 000 000, la carboxyméthylcellulose sodique réticulée, le carboxyméthylamidon et ses sels, le copolymère divinylbenzène-méthacrylate de potassium.

4. Comprimé selon l'une des revendications 2 ou 3, caractérisé en ce que les excipients hydrophiles constituent de 1 à 50% et de préférence de 10 à 30% du poids de la couche.

5. Comprimé selon l'une quelconque des revendications 1 à 4, caractérisé en ce que la couche 3 a une composition identique à celle de la première couche 1 et les mêmes propriétés fonctionnelles.

6. Comprimé selon la revendication 5, caractérisé en ce que les couches 1 et 3 diffèrent par la quantité appliquée sur la couche 2 et leur épaisseur.

7. Comprimé selon l'une quelconque des revendications 1 à 6, caractérisé en ce qu'au contact avec des liquides aqueux, au moins une des couches du comprimé augmente d'au moins 1,5 et de préférence d'au moins 3 fois par rapport au volume initial au bout de deux heures.

8. Comprimé selon l'une quelconque des revendications 1 à 7, caractérisé en ce que les substances polymères hydrophiles sont choisies dans le groupe comprenant l'hydroxyméthylcellulose, l'hydroxyéthylcellulose, l'hydroxypropylméthylcellulose ayant un poids moléculaire de 1000 à 4.000.000, l'hydroxypropylcellulose ayant un poids moléculaire de 2.000 à 2.000.000, les carboxyvinylpolymères, les chitosanes, les mannanes, les galactomannanes, les xanthanes, les carraghènanes, l'amylose, l'acide alginique, ses sels et ses dérivés, les pectines, les acrylates, les méthacrylates, les copolymères acryliques/méthacryliques, les polyanhydrides, les polyaminoacides, les poly(méthyl vinyl éthers/anhydride maléique), les alcools polyvinyliques, les glucanes, les scléroglucanes, la carboxyméthylcellulose et ses dérivés, l'éthylcellulose, la méthylcellulose et, en général, les dérivés cellulosiques hydrophiles.

9. Comprimé selon la revendication 1, caractérisé en ce que la couche 2 contenant la substance active est constituée de 5 à 90 %, de préférence de 10 à 85 %, en poids de polymères hydrophiles.

10. Comprimé selon l'une quelconque des revendications 1 à 9, caractérisé en ce que la troisième couche contient du chlorhydrate d'alfuzosine.

11. Comprimé selon l'une quelconque des revendications 1 à 10, caractérisé en ce que la quantité de chlorhydrate d'alfuzosine véhiculée dans le comprimé va de 2,5 à 50 mg.

12. Comprimé selon l'une quelconque des revendications 1 à 11, caractérisé en ce que pour favoriser le gonflement rapide d'une des couches on utilise des substances du groupe des tensio-actifs tels que le laurylsulfate de sodium, le ricinoléate de sodium, le tétradécylsulfate de sodium, le dioctylsulfosuccinate de sodium, le cétomacrogol, le poloxamère, le monostéarate de glycérol, les polysorbates, le sorbitanemonolaurate, les lécithines ou tout autre tensio-actif pharmaceutiquement acceptable.

13. Comprimé selon l'une quelconque des revendications 1 à 12, caractérisé en ce que pour favoriser la pénétration de l'eau et/ou des fluides aqueux dans les différentes couches, on utilise des diluants hydrophiles tels que le mannitol, le lactose, des amidons d'origines différentes, le sorbitol, le xylitol, la cellulose microcristalline et/ou en général des substances qui favorisent la pénétration de l'eau dans une préparation.

14. Comprimé selon l'une quelconque des revendications 1 à 13, caractérisé en ce que pour ralentir la pénétration de l'eau et/ou des fluides aqueux dans la couche 2 contenant la substance active et dans la ou les couches 1 et 3, on utilise des diluants hydrophobes tels que le monostéarate de glycérol, les palmitates, les huiles végétales hydrogénées, des cires, des glycérides mono, bi ou trisubstitués.

15. Comprimé selon l'une quelconque des revendications 1 à 14, caractérisé par le fait que les différentes couches du comprimé peuvent avoir des épaisseurs différentes allant de 0,2 mm à 8 mm, de préférence de 1 mm à 4 mm.

16. Comprimé selon l'une quelconque des revendications 1 à 15, caractérisé en ce que la pression appliquée pour obtenir le comprimé varie de 7 à 50 KN.

17. Comprimé selon l'une quelconque des revendications 1 à 16, caractérisé en ce qu'il est recouvert d'un enrobage pouvant comporter du chlorhydrate d'alfuzosine.

18. Composition pharmaceutique comprenant un ou plusieurs comprimés selon l'une quelconque des revendications 1 à 17.

19. Comprimé selon la revendication 1, caractérisé en ce que:
a) la première couche 1 a la composition suivante en pourcentage :
| | |
|---|---|
| Hydroxypropylméthylcellulose | 79,75 % |
| Huile de ricin hydrogénée | 13,50 % |
| Oxyde de fer jaune | 0,25 % |
| Ethylcellulose | 5,00 % |
| Stéarate de magnésium | 1,00 % |
| Gel de silice | 0,50 % |
b) la deuxième couche 2 a la composition unitaire suivante :
| | |
|---|---|
| Chlorhydrate d'alfuzosine | 10,00 mg |
| Mannitol | 10,00 mg |
| Hydroxypropylméthylcellulose USP 2208 | 10,00 mg |
| Polyvinylpyrrolidone | 3,20 mg |
| Cellulose microcristalline | 65,00 mg |
| Stéarate de magnésium | 1,00 mg |
| Silice colloïdale | 1,25 mg |
et
c) la troisième couche 3 a la composition suivante en pourcentage :
| | |
|---|---|
| Hydroxypropylméthylcellulose | 76,00 % |
| Huile de ricin hydrogénée | 18,60 % |
| Polyvinylpyrrolidone | 3,15 % |
| Oxyde de fer jaune | 0,10 % |
| Stéarate de magnésium | 0,70 % |
| Silice colloïdale | 1,45 %. |

20. Comprimé selon la revendication 19, caractérisé en ce que la première couche 1 a la composition suivante en pourcentage:
| | |
|---|---|
| Hydroxypropylméthylcellulose | 75,00 % |
| Béhénate de glycérol | 13,40 % |
| Polyvinylpyrrolidone | 5,00 % |
| Oxyde de fer | 0,10 % |
| Polyvinylpyrrolidone | 5,00 % |
| Stéarate de magnésium | 1,00 % |
| Silice colloïdale | 0,50 %. |

21. Comprimé selon la revendication 1 caractérisé en ce que: la première couche 1 et troisième couche 3 ont la composition suivante en pourcentage:
| | |
|---|---|
| Hydroxypropylméthylcellulose USP 2208 | 40,00 % |
| Lactose | 39,75 % |
| Béhénate de glycérol | 13,50 % |
| Oxyde de fer jaune | 0,25 % |
| Polyvinylpyrrolidone | 5,00 % |
| Stéarate de magnésium | 1,00 % |
| Silice colloïdale | 0,50 % |
et la deuxième couche 2 a la composition unitaire suivante :
| | |
|---|---|
| Chlorhydrate d'alfuzosine | 10,00 mg |
| Lactose | 60,30 mg |
| Hydroxypropylméthylcellulose USP 2208 | 25,00 mg |
| Polyvinylpyrrolidone | 3,20 mg |
| Stéarate de magnésium | 1,00 mg |
| Silice colloïdale | 0,50 mg. |

22. Comprimé selon la revendication 21, caractérisé en ce que la deuxième couche 2 a la composition unitaire suivante :
| | |
|---|---|
| Chlorhydrate d'alfuzosine | 15,00 mg |
| Lactose | 55,30 mg |
| Hydroxypropylméthylcellulose USP 2208 | 25,00 mg |
| Polyvinylpyrrolidone | 3,20 mg |
| Stéarate de magnésium | 1,00 mg |
| Silice colloïdale | 0,50 mg. |

## Patentansprüche

1. Oral zu verabreichende pharmazeutische Tablette zur gesteuerten Freisetzung von Alfuzosin-Hydrochlorid im Bereich der proximalen Segmente des Magen-Darm-Trakts, welche Tablette gekennzeichnet ist durch:
a) eine erste Schicht 1 mit der Eigenschaft, im Kontakt mit wäßrigen biologischen Flüssigkeiten in starkem Maße und schnell anzuschwellen, welche Schicht durch Verpressen einer Mischung oder eines Granulats, die bzw. das hydrophile Polymere enthält, die 5,0 bis 90 % und vorzugsweise 10 bis 85 Gew.-% der Schicht ausmachen, gebildet worden ist,
b) eine der ersten Schicht benachbarte oder sie überlagende zweite Schicht 2, in der Alfuzosin-Hydrochlorid in einem Trägermaterial vorliegt, wobei diese Schicht mit hydrophilen Polymeren und anderen Hilfsstoffen formuliert ist, um dem Präparat geeignete Kompressibilitätseigenschaften zu verleihen und die Freisetzung des Alfuzosin-Hydrochlorids innerhalb eines vorbestimmten Zeitintervalls zu ermöglichen,
c) und gegebenenfalls eine durch Verpressen erzeugte und auf der Schicht 2 angeordnete dritte Schicht 3, die im allgemeinen insbesondere aus hydrophilen Polymeren gebildet ist, die gelieren und/oder anschwellen und gegebenenfalls erodieren können und eine Sperr-Funktion besitzen, welche die Freisetzung des Alfuzosin-Hydrochlorids der Schicht 2 moduliert, wobei die Schicht 3 zunächst für den Durchtritt des Wirkstoffs sehr wenig durchlässig ist.

2. Tablette nach Anspruch 1, dadurch gekennzeichnet, daß zur Begünstigung eines schnellen Anschwellens mindestens eine der Schichten aus hydrophilen Produkten und/oder Trägermaterialien gebildet ist.

3. Tablette nach Anspruch 2, dadurch gekennzeichnet, daß die hydrophilen Trägermterialien der Gruppe der "Supersprengmittel" angehören, die vernetztes Polyvinylpyrrolidon, Hydroxypropylcellulose und Hydroxypropylmethylcellulose mit einem Molekulargewicht von 1000 bis 1 000 000, vernetzte Natriumcarboxymethylcellulose, Carboxymethylstärke und deren Salze und Divinylbenzl-Kaliummethacrylat-Copolymere umfaßt.

4. Tablette nach einem der Ansprüche 2 oder 3, dadurch gekennzeichnet, daß die hydrophilen Trägermaterialien 1 bis 50 %, vorzugsweise 10 bis 30 Gew.-% der Schicht ausmachen.

5. Tablette nach einem der Ansprüche 1 bis 4, dadurch gekennzeichnet, daß die Schicht 3 eine Zusammensetzung besitzt, die mit der der ersten Schicht 1 identisch ist und die die gleichen funktionellen Eigenschaften aufweist.

6. Tablette nach Anspruch 5, dadurch gekennzeichnet, daß die Schichten 1 und 3 sich durch die auf die Schicht 2 aufgetragene Menge und ihre Dicke unterscheiden.

7. Tablette nach einem der Ansprüche 1 bis 6, dadurch gekennzeichnet, daß mindestens eine der Schichten der Tablette im Kontakt mit wäßrigen Flüssigkeiten nach zwei Stunden im Vergleich zu dem Anfangsvolumen um das mindestens 1,5-fache und vorzugsweise mindestens 3-fache zunimmt.

8. Tablette nach einem der Ansprüche 1 bis 7, dadurch gekennzeichnet, daß die hydrophilen polymeren Substanzen aus der Gruppe ausgewählt sind, die Hydroxymethylcellulose, Hydroxyethylcellulose, Hydroxypropylmethylcellulose mit einem Molekulargewicht von 1000 bis 4.000.000, Hydroxypropylcellulose mit einem Molekulargewicht von 2.000 bis 2.000.000, Carboxyvinylpolymere, Chitosane, Mannane, Galactomannane, Xanthane, Carragheenane, Amylose, Alginsäure, deren Salze und Derivate, Pectine, Acrylate, Methacrylate, Acryl/Methacryl-Copolymere, Polyanhydride, Polyaminosäuren, Poly(methylvinylether/Maleinsäureanhydrid), Polyvinylalkohole, Glucane, Scleroglucane, Carboxymethylcellulose und deren Derivate, Ethylcellulose, Methylcellulose und im allgemeinen hydrophile Cellulosederivate umfaßt.

9. Tablette nach Anspruch 1, dadurch gekennzeichnet, daß die Schicht 2, welche den Wirkstoff enthält, aus 5 bis 90 %, vorzugsweise 10 bis 85 Gew.-% hydrophilen Polymeren gebildet ist.

10. Tablette nach einem der Ansprüche 1 bis 9, dadurch gekennzeichnet, daß die dritte Schicht Alfuzosin-Hydrochlorid enthält.

11. Tablette nach einem der Ansprüche 1 bis 10, dadurch gekennzeichnet, daß die mit dem Trägermaterial in der Tablette vorhandene Menge an Alfuzosin-Hydrochlorid 2,5 bis 50 mg beträgt.

12. Tablette nach einem der Ansprüche 1 bis 11, dadurch gekennzeichnet, daß man zur Begünstigung des schnellen Anschwellens einer der Schichten Substanzen aus der Gruppe der oberflächenaktiven Mittel, wie Natriumlaurylsulfat, Natriumricinoleat, Natriumtetradecylsulfat, Natriumdioctylsulfosuccinat, Cetomacrogol, Poloxamer, Glycerol-monostearat, Polysorbate, Sorbitanmonolaurat, Lecithine oder irgendwelche anderen pharmazeutisch annehmbaren oberflächenaktiven Mittel verwendet.

13. Tablette nach einem der Ansprüche 1 bis 12, dadurch gekennzeichnet, daß man zur Begünstigung der Penetration der verschiedenen Schichten mit Wasser und/oder wäßrigen Fluiden hydrophile Verdünnungsmittel verwendet, wie Mannitol, Lactose, Stärken unterschiedlichen Ursprungs, Sorbitol, Xylitol, mikrokristalline Cellulose und/oder im allgemeinen Substanzen, welche die Penetration von Wasser in ein Präparat begünstigen.

14. Tablette nach einem der Ansprüche 1 bis 13, dadurch gekennzeichnet, daß man zur Verlangsamung der Penetration von Wasser und/oder wäßrigen Fluiden in die den Wirkstoff enthaltende Schicht 2 und in die Schichten 1 und/oder 3 hydrophobe Verdünnungsmittel verwendet, wie Glycerolmonostearat, Palmitate, hydrierte pflanzliche Öle, Wachse und mono-, di- oder trisubstituierte Glyceride.

15. Tablette nach einem der Ansprüche 1 bis 14, dadurch gekennzeichnet, daß die verschiedenen Schichten der Tablette unterschiedliche Dicken aufweisen können, die sich von 0,2 mm bis 8 mm, vorzugsweise von 1 mm bis 4 mm erstrecken.

16. Tablette nach einem der Ansprüche 1 bis 15, dadurch gekennzeichnet, daß der für die Erzeugung der Tablette angewandte Druck 7 bis 50 kN beträgt.

17. Tablette nach einem der Ansprüche 1 bis 16, dadurch gekennzeichnet, daß sie mit einer Umhüllung bedeckt ist, die Alfuzosin-Hydrochlorid enthalten kann.

18. Pharmazeutische Zubereitung umfassend eine oder mehrere Tabletten nach einem der Ansprüche 1 bis 17.

19. Tablette nach der Anspruch 1, dadurch gekennzeichnet, daß:
a) die erste Schicht 1 die folgende prozentuale Zusammensetzung besitzt:
| | |
|---|---|
| Hydroxypropylmethylcellulose | 79,75 % |
| hydriertes Rizinusöl | 13,50 % |
| gelbes Eisenoxid | 0,25 % |
| Ethylcellulose | 5,00 % |
| Magnesiumstearat | 1,00 % |
| Kieselgel | 0,50 % |
b) die zweite Schicht 2 die folgende einheitliche Zusammensetzung besitzt:
| | |
|---|---|
| Alfuzosin-Hydrochlorid | 10,00 mg |
| Mannitol | 10,00 mg |
| Hydroxypropylmethylcellulose USP 2208 | 10,00 mg |
| Polyvinylpyrrolidon | 3,20 mg |
| mikrokristalline Cellulose | 65,00 mg |
| Magnesiumstearat | 1,00 mg |
| kolloidales Siliciumdioxid | 1,25 mg |
und
c) die dritte Schicht 3 die folgende prozentuale Zusammensetzung besitzt:
| | |
|---|---|
| Hydroxypropylmethylcellulose | 76,00 % |
| hydriertes Rizinusöl | 18,60 % |
| Polyvinylpyrrolidon | 3,15 % |
| gelbes Eisenoxid | 0,10 % |
| Magnesiumstearat | 0,70 % |
| kolloidales Siliciumdioxid | 1,45 %. |

20. Tablette nach Anspruch 19, dadurch gekennzeichnet, daß die erste Schicht 1 die folgende prozentuale Zusammensetzung besitzt:
| | |
|---|---|
| Hydroxypropylmethylcellulose | 75,00 % |
| Glycerol-behenat | 13,40 % |
| Polyvinylpyrrolidon | 5,00 % |
| Eisenoxid | 0,10 % |
| Polyvinylpyrrolidon | 5,00 % |
| Magnesiumstearat | 1,00 % |
| kolloidales Siliciumdioxid | 0,50 %. |

21. Tablette nach Anspruch 1, dadurch gekennzeichnet, daß die erste Schicht 1 und die dritte Schicht 3 die folgende prozentuale Zusammensetzung besitzen:
| | |
|---|---|
| Hydroxypropylmethylcellulose USP 2208 | 40,00 % |
| Lactose | 39,75 % |
| Glycerol-behenat | 13,50 % |
| gelbes Eisenoxid | 0,25 % |
| Polyvinylpyrrolidon | 5,00 % |
| Magnesiumstearat | 1,00 % |
| kolloidales Siliciumdioxid | 0,50 % |
und die zweite Schicht 2 die folgende einheitliche Zusammensetzung besitzt:
| | |
|---|---|
| Alfuzosin-Hydrochlorid | 10,00 mg |
| Lactose | 60,30 mg |
| Hydroxypropylmethylcellulose USP 2208 | 25,00 mg |
| Polyvinylpyrrolidon | 3,20 mg |
| Magnesiumstearat | 1,00 mg |
| kolloidales Siliciumdioxid | 0,50 mg. |

22. Tablette nach Anspruch 21, dadurch gekennzeichnet, daß die zweite Schicht 2 die folgende einheitliche Zusammensetzung besitzt:
| | |
|---|---|
| Alfuzosin-Hydrochlorid | 15,00 mg |
| Lactose | 55,30 mg |
| Hydroxypropylmethylcellulose USP 2208 | 25,00 mg |
| Polyvinylpyrrolidon | 3,20 mg |
| Magnesiumstearat | 1,00 mg |
| kolloidales Siliciumdioxid | 0,50 mg. |

## Claims

1. Pharmaceutical tablet intended for the oral route, for the controlled release of alfuzosin hydrochloride into the proximal segments of the gastrointestinal tract, this tablet being characterized in that it consists of:
a) a first layer 1 having the property of swelling considerably and quickly on contact with aqueous biological fluids, the said layer being produced by compression of a mixture or of a granulate comprising hydrophilic polymers constituting from 5.0 to 90% and preferably from 10 to 85% of the weight of the layer,
b) a second layer 2 adjacent to or superimposed on the first layer, in which the alfuzosin hydrochloride is conveyed, this layer being formulated with hydrophilic polymers and with other auxiliary substances, in order to give the preparation suitable properties of compressibility and in order to allow the release of alfuzosin hydrochloride within a predetermined time period,
c) and optionally a third layer 3 obtained by compression and applied to the layer 2, generally consisting in particular of hydrophilic polymers which gel and/or swell and which may then optionally be broken down and having a barrier function which modifies the release of the alfuzosin hydrochloride from the layer 2, the layer 3 being primarily highly impervious to passage of the active substance.

2. Tablet according to Claim 1, characterized in that in order to promote rapid swelling, at least one of the layers consists of hydrophilic products and/or excipients.

3. Tablet according to Claim 2, characterized in that the hydrophilic excipients belong to the group of "super disintegrating" excipients comprising crosslinked polyvinylpyrrolidone, hydroxypropylcellulose and hydroxypropylmethylcellulose having a molecular weight of from 1000 to 1,000,000, crosslinked sodium carboxymethylcellulose, carboxymethyl starch and its salts, and divinylbenzene/potassium methacrylate copolymer.

4. Tablet according to either of Claims 2 and 3, characterized in that the hydrophilic excipients constitute from 1 to 50% and preferably from 10 to 30% of the weight of the layer.

5. Tablet according to any one of Claims 1 to 4, characterized in that the layer 3 has an identical composition to that of the first layer 1 and the same functional properties.

6. Tablet according to Claim 5, characterized in that the layers 1 and 3 differ in the amount applied to the layer 2 and their thickness.

7. Tablet according to any one of Claims 1 to 6, characterized in that, on contact with aqueous liquids, at least one of the layers of the tablet increases by at least 1.5 and preferably at least 3 times relative to the initial volume, after two hours.

8. Tablet according to any one of Claims 1 to 7, characterized in that the hydrophilic polymer substances are chosen from the group comprising hydroxymethylcellulose, hydroxyethylcellulose, hydroxypropylmethylcellulose having a molecular weight of from 1000 to 4,000,000, hydroxypropylcellulose having a molecular weight of from 2000 to 2,000,000, carboxyvinyl polymers, chitosans, mannans, galactomannans, xanthans, carrageenans, amylose, alginic acid, its salts and its derivatives, pectins, acrylates, methacrylates, acrylic/methacrylic copolymers, polyanhydrides, polyamino acids, poly(methyl vinyl ethers/maleic anhydride) polymers, polyvinyl alcohols, glucans, scleroglucans, carboxymethylcellulose and its derivatives, ethylcellulose, methylcellulose and, in general, hydrophilic cellulose derivatives.

9. Tablet according to Claim 1, characterized in that the layer 2 containing the active substance consists of 5 to 90%, preferably 10 to 85%, by weight of hydrophilic polymers.

10. Tablet according to any one of Claims 1 to 9, characterized in that the third layer contains alfuzosin hydrochloride.

11. Tablet according to any one of Claims 1 to 10, characterized in that the amount of alfuzosin hydrochloride carried in the tablet ranges from 2.5 to 50 mg.

12. Tablet according to any one of Claims 1 to 11, characterized in that substances from the group of surfactants such as sodium lauryl sulphate, sodium ricinoleate, sodium tetradecyl sulphate, sodium dioctyl sulphosuccinate, cetomacrogol, poloxamer, glyceryl monostearate, polysorbates, sorbitan monolaurate, lecithins or any other pharmaceutically acceptable surfactant, are used in order to promote rapid swelling of one of the layers.

13. Tablet according to any one of Claims 1 to 12, characterized in that hydrophilic diluents such as mannitol, lactose, starches of various origins, sorbitol, xylitol, microcrystalline cellulose and/or in general, substances which promote the penetration of water into a preparation are used in order to promote the penetration of water and/or aqueous fluids into the various layers.

14. Tablet according to any one of Claims 1 to 13, characterized in that hydrophobic diluents such as glyceryl monostearate, palmitates, hydrogenated vegetable oils, waxes, mono-, di- or trisubstituted glycerides are used for slowing down the penetration of water and/or of aqueous fluids into the layer 2 containing the active substance and into the layer(s) 1 and 3.

15. Tablet according to any one of Claims 1 to 14, characterized in that the various layers of the tablet may have different thicknesses ranging from 0.2 mm to 8 mm, preferably from 1 mm to 4 mm.

16. Tablet according to any one of Claims 1 to 15, characterized in that the pressure applied in order to obtain the tablet ranges from 7 to 50 KN.

17. Tablet according to any one of Claims 1 to 16, characterized in that it is covered with a coating which may contain alfuzosin hydrochloride.

18. Pharmaceutical composition comprising one or more tablets according to any one of Claims 1 to 17.

19. Tablet according to Claim 1, characterized in that:
a) the first layer 1 has the following percentage composition:
| | |
|---|---|
| Hydroxypropylmethylcellulose | 79.75% |
| Hydrogenated castor oil | 13.50% |
| Yellow iron oxide | 0.25% |
| Ethylcellulose | 5.00% |
| Magnesium stearate | 1.00% |
| Silica gel | 0.50% |
b) the second layer 2 has the following unit composition:
| | |
|---|---|
| Alfuzosin hydrochloride | 10.00 mg |
| Mannitol | 10.00 mg |
| Hydroxypropylmethylcellulose USP 2208 | 10.00 mg |
| Polyvinylpyrrolidone | 3.20 mg |
| Microcrystalline cellulose | 65.00 mg |
| Magnesium stearate | 1.00 mg |
| Colloidal silica | 1.25 mg |
and
c) the third layer 3 has the following percentage composition:
| | |
|---|---|
| Hydroxypropylmethylcellulose | 76.00% |
| Hydrogenated castor oil | 18.60% |
| Polyvinylpyrrolidone | 3.15% |
| Yellow iron oxide | 0.10% |
| Magnesium stearate | 0.70% |
| Colloidal silica | 1.45%. |

20. Tablet according to Claim 19, characterized in that the first layer 1 has the following percentage composition:
| | |
|---|---|
| Hyrdroxypropylmethylcellulose | 75.00% |
| Glyceryl behenate | 13.40% |
| Polyvinylpyrrolidone | 5.00% |
| Iron oxide | 0.10% |
| Polyvinylpyrrolidone | 5.00% |
| Magnesium stearate | 1.00% |
| Colloidal silica | 0.50%. |

21. Tablet according to Claim 1, characterized in that:
the first layer 1 and third layer 3 have the following percentage composition:
| | |
|---|---|
| Hydroxypropylmethylcellulose USP 2208 | 40.00% |
| Lactose | 39.75% |
| Glyceryl behenate | 13.50% |
| Yellow iron oxide | 0.25% |
| Polyvinylpyrollidone | 5.00% |
| Magnesium stearate | 1.00% |
| Colloidal silica | 0.50% |
and the second layer 2 has the following unit composition:
| | |
|---|---|
| Alfuzosin hydrochloride | 10.00 mg |
| Lactose | 60.30 mg |
| Hydroxypropylmethylcellulose USP 2208 | 25.00 mg |
| Polyvinylpyrrolidone | 3.20 mg |
| Magnesium stearate | 1.00 mg |
| Colloidal silica | 0.50 mg |

22. Tablet according to Claim 21, characterized in that the second layer 2 has the following unit composition:
| | |
|---|---|
| Alfuzosin hydrochloride | 15.00 mg |
| Lactose | 55.30 mg |
| Hydroxypropylmethylcellulose USP 2208 | 25.00 mg |
| Polyvinylpyrrolidone | 3.20 mg |
| Magnesium stearate | 1.00 mg |
| Colloidal silica | 0.50 mg. |
